# EUROPEAN PATENT SPECIFICATION

(11) **EP 0 956 028 B1**
(45) Date of publication and mention of the grant of the patent: **12.09.2001**
(21) Application number: 97949718.7
(22) Date of filing: 10.12.1997
(51) Int. Cl.: A61K 35/28, A61K 35/22, A61K 35/44, C12N 5/00

(54) **SUBMUCOSAL TISSUE INHIBITION OF NEOPLASTIC CELL GROWTH**
DURCH SUBMUKOSALES GEWEBE ERZEUGTE INHIBIERUNG DES NEOPLASTISCHEN ZELLWACHSTUMS
INHIBITION DE LA CROISSANCE DE CELLULES NEOPLASIQUES PAR DES TISSUS SOUS-MUQUEUX

(30) Priority: 10.12.1996 US 32681 P
(43) Date of publication of application: 17.11.1999
(73) Proprietor: Purdue Research Foundation, West Lafayette, IN 47906 (US); Methodist Health Group, Inc., Indianapolis, IN 46206-1367 (US)
(72) Inventor: CRITSER, John, K., Carmel, IN 46033 (US); LIU, Chi, Indianapolis, IN 46228 (US); DEMETER, Robert, J., Mooresville, IN 46158 (US)
(74) Representative: Bannerman, David Gardner
(86) International application number: US9722039
(87) International publication number: WO9825635

(56) References cited:
- WO-A-96/24661
- US-A- 4 902 508

## Description

### Field of the Invention

The present invention relates to the use of submucosal tissue of a warm blooded vertebrate for the preparation of a pharmaceutical composition for inhibiting the growth of undifferentiated cells.

### Background and Summary

Traditionally cancer has been treated by surgery, radiation, chemotherapy or a combination of any one of these treatments. However, some cancers are difficult to treat using standard surgical methods due to the location or composition of the neoplastic cells, whereas anti-tumor therapies based on chemotherapy and/or radiation treatments can be extremely debilitating to the patient. Accordingly, an anti-cancer therapy is desired that is effective in neutralizing neoplastic cell growth while having a minimal impact on normal cells.

The present invention is directed to the use of vertebrate submucosa-derived matrices as pharmaceutical agents to inhibit the growth of undifferentiated cells. The collagenous matrices for use in accordance with the present invention comprise highly conserved collagens, glycoproteins, proteoglycans, and glycosaminoglycans in their natural configuration and natural concentration. The extracellular collagenous matrix for use in this invention is derived from submucosal tissue of a warm-blooded vertebrate.

Submucosal tissue can be obtained from various tissue sources, harvested from animals raised for meat production, including, for example, pigs, cattle and sheep or other warm-blooded vertebrates. More particularly, the submucosa is isolated from warm-blooded tissues including the alimentary, respiratory, intestinal, urinary or genital tracts of warm-blooded vertebrates. In general submucosa is prepared from these tissue sources by delaminating the submucosal from both the smooth muscle layers and the mucosai layers. The preparation of intestinal submucosa is described and claimed in U.S. Patent No. 4,902,508. Urinary bladder submucosa and its preparation is described in U.S. Patent No. 5,554,389.
Stomach submucosa has also been obtained and characterized using similar tissue processing techniques. Such is described in U.S. patent application No. 60/032,683 titled STOMACH SUBMUCOSA DERIVED TISSUE GRAFT, fled on December 10, 1996. Briefly, stomach submucosa is prepared from a segment of stomach in a procedure similar to the preparation of intestinal submucosa. A segment of stomach tissue is first subjected to abrasion using a longitudinal wiping motion to remove the outer layers (particularly the smooth muscle layers) and the luminal portions of the tunica mucosa layers. The resulting submucosa tissue has a thickness of about 100 micrometers, and consists primarily (greater than 98%) of acellular, eosinophilic staining (H&E stain) extracellular matrix material.

Submucosal tissue can be used in either its natural configuration or in a comminuted or partially digested fluidized form. Vertebrate submucosal tissue is a plentiful by-product of commercial meat production operations and is thus a low cost material, especially when the submucosal tissue is used in its native layer sheet configuration. Submucosal tissues for use in accordance with this invention include intestinal submucosa, stomach submucosa, urinary bladder submucosa, and uterine submucosa. Intestinal submucosal tissue is one preferred material, and more particularly small intestinal submucosa.

Suitable intestinal submuccsal tissue typically comprises the tunica submucosa delaminated from both the tunica muscularis and at least the luminal portion of the tunica mucosa. In one embodiment of the present invention the intestinal submucosal tissue comprises the tunica submucosa and basilar portions of the tunica mucosa including the lamina muscularis mucosa and the stratum compactum which layers are known to vary thickness and in definition dependent on the source vertebrate species.

It has been well documented that submucosal tissue is capable of inducing host tissue proliferation, and remodeling and regeneration of appropriate tissue structures upon implantation in a number of microenvironments *in vivo* (e.g., tendon, ligament, bone, articular cartilage, artery, and vein). The use of such tissue in sheet form and fluidized forms for inducing the formation of endogenous tissues is described and claimed in U.S. Patent Nos. 5,281,422 and 5,275,826.

Applicants have made the surprising discovery that in addition to submucosal tissue's ability to induce the growth of determined/differentiated cell populations, submucosal tissue will inhibit the growth of undifferentiated cells. In particular, the submucosal ussue compositions of the present invention can be utilized to kill neoplastic cells. Therefore it is anticipated that the submucosal tissue composition of the present invention can be utilized to perform two simultaneous functions: the inhibition of neoplastic cell growth and the induction of normal host tissue growth.

In accordance with the present invention submucosal tissue of a warm blooded vertebrate is used for the preparation of a pharmaceutical composition for inhibiting the growth of undifferentiated cells or for preparing a pharmaceutical composition for killing the undifferentiated cells. More particularly the pharmaceutical compositions will have anti-tumor activity, The pharmaceutical compositions will be utilized in a method of inhibiting the growth of undifferentiated cells *in vivo*. The method comprises the step of contacting the undifferentiated cells with an amount of submucosal tissue effective to inhibit the growth of the cells.

### Detailed Description of the Preferred Embodiments

There is provided in accordance with this invention a method for suppressing the proliferation and growth of undifferentiated eukaryotic cells. In particular the present invention is directed to the inhibition of neoplastic cell growth. Generally the method comprises the step of contacting the neoplastic eukaryotic cells with a vertebrate submucosa-derived collagenous matrix. The term "neoplastic cell" is defined herein to include any eukaryotic cell or group of eukaryotic cells that proliferate/grow without control and perform no physiological function. The term "contacting" as used herein is intended to include both direct and indirect contact, for example in fluid communication, of the submucosa-derived collagenous matrix and the neoplastic cells.

In one embodiment, the submucosa-derived collagenous matrix (submucosal tissue) comprises tunica submucosa delaminated from both the tunica muscuiaris and at least the iuminal portion of the tunica mucosa of intestinal tissue of a warm-blooded vertebrate. The preparation of intestinal submucosal tissue for use in accordance with this invention is described in U.S. Patent No. 4,902,508. A segment of vertebrate intestine, preferably harvested from porcine, ovine or bovine species, but not excluding other species, is subjected to abrasion using a longitudinal wiping motion to remove the outer layers, comprising smooth muscle tissues, and the innermost layer, *i.e.*, the luminal portion of the tunica mucosa. The submucosal tissue is rinsed with saline, optionally sterilized, and can be stored in a hydrated or dehydrated state. Lyophilized or air dried submucosa tissue can be rehydrated and used in accordance with this invention without significant loss of its cell proliferative activity and, presumably without loss of its inhibitory effect on undifferentiated cells.

The graft compositions of the present invention can be sterilized using conventional sterilization techniques including glutaraldehyde tanning, formaldehyde tanning at acidic pH, propylene oxide treatment, gas plasma sterilization, gamma radiation, electron beam, peracetic acid sterilization. Sterilization techniques which do not adversely affect the mechanical strength, structure, and biotropic properties of the submucosal tissue is preferred. For instance, strong gamma radiation may cause loss of strength of the sheets of submucosal tissue. Preferred sterilization techniques include exposing the graft to peracetic acid, 1-4 Mrads gamma irradiation (more preferably 1-2.5 Mrads of gamma irradiation) or gas plasma sterilization; peracetic acid sterilization is the most preferred sterilization method. Typically, the submucosal tissue is subjected to two or more sterilization processes. Accordingly, after the submucosal tissue is first sterilized, for example by chemical treatment, the tissue may be wrapped in a plastic or foil wrap and sterilized again using electron beam or gamma irradiation sterilization techniques.

The submucosal tissue specified for use in accordance with this invention can also be in a fluidized form. Submucosal tissue can be fluidized by comminuting the tissue and optionally subjecting it to enzymatic digestion to form a homogenous solution. The preparation of fluidized forms of submucosa tissue is described in U.S. Patent No. 5,275,826.
Fluidized forms of submucosai tissue are prepared by comminuting submucosa tissue by tearing, cutting, grinding, or shearing the harvested submucosal tissue. Thus pieces of submucosal tissue can be comminuted by shearing in a high speed blender, or by grinding the submucosa in a frozen or freeze-dried state to produce a powder that can thereafter be hydrated with water or a burfered saline to form a submucosal fluid of liquid, gel or paste-like consistency.

The comminuted submucosa formulation can further be treated with an enzymatic composition to provide a homogenous solution of partially solubilized submucosa. The enzymatic composition may comprise one or more enzymes that are capable of breaking the covalent bonds of the structural components of the submucosal tissue. For example, the comminuted submucosal tissue can be treated with a protease, such as trypsin or pepsin at an acidic pH, for a period of time sufficient to solubilize all or a major portion of the submucosal tissue protein components. In addition the submucosal tissue can be treated with a collagenase or glycosaminoglycanase to prepare solubilized forms of submucosal tissue. After treating the comminuted submucosa formulation with the enzymatic composition, the tissue is optionally filtered to provide a homogenous solution.

The viscosity of fluidized submucosa for use in accordance with this invention can be manipulated by controlling the concentration of the submucosa component and the degree of hydration. The viscosity can be adjusted to a range of about 2 to about 300,000 cps at 25 ° C. Higher viscosity formulations, for example, gels, can be prepared from the submucosa digest solutions by adjusting the pH of such solutions to about 6.0 to about 7.4.

It has been well documented that submucosal tissue is capable of inducing host tissue proliferation, remodeling and regeneration of appropriate tissue structures upon implantation in a number of microenvironments *in vivo* (e.g., tendon, ligament, bone, articular cartilage, artery, and vein). The use of such tissue in sheet form and fluidized forms for inducing the formation of endogenous tissues is described and claimed in U.S. Patent Nos. 5,281,422 and 5,275,826. Furthermore, as disclosed herein, compositions comprising submucosal tissue (in both sheet form and fluidized forms) have been found to inhibit the growth or proliferation of undifferentiated eukaryotic cells.

In accordance with the present invention submucosal tissue is used for the preparation of a pharmaceutical composition for inhibiting the growth of neoplastic eukaryotic cells. In one embodiment the submucosal tissue comprises vertebrate intestinal submucosal tissue, more specifically, the tunica submucosa delaminated from both the tunica muscularis and at least the luminal portion of the tunica mucosa. In one preferred embodiment of the present invention the intestinal submucosal tissue comprises the tunica submucosa and basilar portions of the tunica mucosa including the lamina muscularis mucosa and the stratum compactum which layers are known to vary in thickness and in definition dependent on the source vertebrate species. The submucosal tissue can be prepared in a variety of forms in accordance with this invention. The submucosal tissue can be used in its native sheet-like configuration, as a fluidized tissue composition, or as a dried powder.

Furthermore, the submucosal tissue compositions can be combined with known chemotherapeutic agents or cytotoxic agents to enhance the effectiveness of currently available chemotherapeutic regiments to inhibit or kill neoplastic cells. In particular the submucosal tissue can be loaded or covalently bonded to chemotherapeutic agents as a means of localizing the chemotherapeutic activity to the site of implantation of the submucosal tissue.

The described submucosal tissue compositions can be utilized in a method of treating a patient having cancer.
The method comprises the step of contacting the neoplastic cells with an amount of submucosal tissue effective to inhibit the growth of the neoplastic cells. For solid tumor masses, the neoplastic cells can be directly contacted with the submucosal compositions by surgical implantation of the submucosal tissue adjacent to or surrounding the neoplastic cells. Alternatively, a less invasive approach can be utilized wherein a pharmaceutically acceptable composition comprising fluidized submucosal tissue is injected directly into or adjacent to the tumor cell mass. For example fluidized submucosal tissue can also be injected into locations that are difficult to reach using standard surgical procedures. Neoplastic cells can also be treated indirectly by administering pharmaceutically acceptable compositions comprising fluidized submucosal tissue by injection into the lymphatic or circulatory system. For example compositions comprising fluidized submucosal tissue can be administered intravenously into a patient having cancer.

Many tumors are difficult to remove by surgical means due to the location or the form of the tumor cells. One common strategy is to remove as much of a tumor mass as possible through surgical means and then rely on chemotherapy and radiation treatments to control or kill the remaining tumor cells.
The excised tumor mass can be replaced with the described submucosal compositions.

It is anticipated that the submucosal tissue compositions will function to both inhibit the growth of the remaining neoplastic cells while inducing the growth of endogenous tissues to assist healing. The submucosal tissue is administered in an amount effective to inhibit the growth of neoplastic cell growth or to kill the neoplastic cells.

After surgical removal of at least a portion of a tumor, a submucosal tissue composition comprising sheets of submucosal tissue formed into a sealed pouch, wherein the pouch contains a fluidized tissue graft composition comprising a suspension of comminuted submucosal tissue or enzymatically digested submucosal tissue can be implanted. The pouch can be sutured in place to hold the submucosal tissue in close proximity to the remaining neoplastic cells. Such a pouch construct can be utilized to replace a tumor mass that has been removed, thus providing support to the surrounding tissues while inducing cellular repair and inhibiting the growth of any remaining tumor cells. For example such a construct could be used to reconstruct soft tissues such as breast tissue, after the removal of tumors.

Fluidized submucosal tissue can also be used to coat prosthetic devices that are implanted to support structures damaged by tumor cell growth. These coated structures would then be capable of inducing host tissue repair while simultaneously inhibiting further neoplastic growth. In particular submucosal tissue coated supports can be implanted to support the spinal column or other bone structures that have been weakened by cancerous growth.

### Example 1

### Inhibition of Tumor Cell Growth by Submucosal Tissue

### Materials and Methods:

Carcinoma ceil lines including A549 (human lung adenocarcinoma), MCF-7 (human breast carcinoma) and human colorectal carcinoma cell lines were obtained from American Type Cell Culture (ATCC). Cell cultures were routinely performed at 37°C in an incubator supplemented with 5% CO and 95% air. Media used were F-12 pius 10° o FBS (fetal bovine serum) (Gibco BRL) for the A549 cells and RPMI plus 10% FBS (Gibco BRL) for both the MCF-7 and colonic carcinoma cell lines. The inhibition of tumor cell growth on several different forms of submucosal tissue including PA-ST (submucosal tissue sterilized in peracetic acid), Native submucosal tissue (no further treatment after dissection), Gamma irradiated submucosal tissue and solubilized submucosal tissue was assessed.

### Culturing of Neoplastic Cells with Submucosal Tissue

Cancer cells were cultured in the presence of various forms of intestinal submucosal tissue at 37°C in an incubator supplemented with 5% CO and 95% air using the following procedures:
1. Direct Contact: Intestinal submucosal tissue and the cultured cells physically contact one another.
2. Indirect Contact: Intestinal submucosal tissue and the cultured cells are separated by a stainless steel mesh.
3. Solubilized intestinal submucosal tissue added to the culture media.
4. Cells are cultured on solubilized intestinal submucosa coated culture plate. The coating was applied by placing 1 ml of solubilized intestinal submucosal tissue in a 35mm culture plate, heating at 37°C for 2 hours, removing the excess intestinal submucosal tissue fluid by aspiration and washing the coated plates once with culture media.

### Sterilization of Submucosal Tissue Before Co-culturing

1. Intestinal submucosal tissue was sterilized by several different means: peracetic acid treatment or gamma irradiation. Gamma irradiated and native submucosal tissue (no further treatment after isolation of the intestinal submucosal tissue) can be used directly as cell culture substrates provided they have been sufficiently rehydrated with the culture media prior to the co-culture (native membranes must be cultured in the presence of antibiotics). Peracetic acid sterilized membranes, were washed to remove residual peracetic acid prior to culturing since peracetic acid residue may be toxic to the cells. Typically peracetic acid sterilized tissues were soaked in a large quality of medium for 24 hours followed by extensive washing with the same medium.
2. Solubilized forms of intestinal submucosal tissue were sterilized by dialyzing against 6.5% chloroform in either 0.1M acetic acid (AA-submucosa) or phosphate buffered saline (PBS-submucosa) for 2 hours at room temperature. The exterior surface of the dialysis tubing is sterilized by rinsing the outside of the tubing with 70% alcohol prior to removal of the intestinal submucosal tissue. The dialysis tubing has a molecular weight cut-off of 12,000-14,000; thus, proteins retained inside tubing are those with molecular weight greater than 14,000.

Tumor cells were plated at a cell density of approximately 5x10⁵ cells/dish of 60 mm tissue culture dishes and grown until they reached about 50% confluence. Cell cultures were then subjected to the following treatments in triplicates: 1) control, cells grown in media only; 2) addition of different quantities of solubilized submucosal tissue (200-800 µl); 3) co-culture with submucosal tissue membranes of different sizes (indirect co-culture). Cell counts were performed 3 days after treatment Tumor cell proliferation on fluidized submucosal tissue coated plates was also compared to that in the control plates.

### Effect of submucosal tissue on the tumor cell proliferation

Culturing tumor cells with submucosal tissue membrane was performed by means of indirect co-culture which allows an easy microscopic evaluation and enzymatic digestion of cells for cell counting. Results showed that submucosal tissue membrane exerted an inhibitory effect on the tumor cell proliferation that was clearly evident after 3 days of co-culture. For A549 human lung carcinoma cells, a significant inhibitory effect (>50% growth inhibition) was observed when more than 0.2 g of submucosal tissue was added to the culture media. The inhibition of cell growth is dose dependent (Table 1). When the concentration of submucosal tissue present in the media was increased to 0.4g (1x1 cm), all of the tumor cells were killed. This amount of submucosal tissue was also lethal to two other tumor cell lines tested (MCF-7 human breast tumor cell line and human colonic carcinoma cell line, see Table 2). All three cell lines normally form adherent cultures *in vitro*. Microscopically, dead cells detached from the culture dishes and were free floating in the medium.

The cytotoxicity of submucosal tissue to the tumor cells is not due to the present of residual peracetic acid since this compound when added exogenously had no significant inhibitory effect on cell proliferation. In addition, native submucosal tissue membrane had a similar inhibitory effect on the growth of cancer cells.

Co-culture with solubilized submucosal tissue, either by addition of solubilized form into the cultures or by growing tumor cells on submucosal tissue coated plates, resulted in only a slight inhibition on tumor cell proliferation (Table 4 and 5). One possible explanation for the lower effect of solubilized submucosal tissue on neoplastic cell growth is that the activity of the active components of the submucosal tissue was inhibited or attenuated by the solubilization procedure. Another possible explanation is that the active components were removed by dialysis. As mentioned above, the proteins retained following dialysis are those with a molecular weight larger than 14,000 dayton (the molecular weight cut off of the dialysis tubing is 12,000-14,000). It is well known that a many growth factors or inhibitors possess a molecular weight around 10,000.

**Table 1.**

| Dose dependent inhibitory effect of submucosal tissue membrane on the proliferation of A549 human lung carcinoma cells | | |
|---|---|---|
| | Cell Count (x10⁵) (day 2 of treatment) | % of Control |
| Control | 19.35 ± 1.23 | 100% |
| 100 µl of Peracidic Acid | 18.81 ± 2.59 | 97% |
| 0.035 g of submucosal tissue | 19.05 ± 0.02 | 98% |
| 0.070 g of submucosal tissue | 15.95 ± 0.23 | 82% |
| 0.167 g of submucosal tissue | 10.94 ± 0.88 | 57% |
| 0.192 g of submucosal tissue | 10.12 ± 0.16 | 52% |
| 0.400 g of submucosal tissue | 0 | 0 |
| 0.700 g of submucosal tissue | 0 | 0 |

**Table 2.**

| Effect of solubilized submucosal tissue on the proliferation of A549 human lung carcinoma cells | | |
|---|---|---|
| | No. of cells x10⁴ at day 4 | |
| | 100 µl | 400 µl |
| control (AA) | 68.4 ± 5.7 | 40.3 ± 6.7 |
| AA-submucosa | 40.4 ± 4.9 | 32.8 ± 12 |

**Table 3.**

| Effect of submucosal tissue coating on the proliferation of A549 human lung carcinoma cells | |
|---|---|
| | No of Cells x 10⁴ at day 4 |
| PBS coated | 29.9 ± 1.80 |
| PBS-submucosa coated | 30.8 ± 2.74 |
| AA coated | 32.0 ± 0.57 |
| AA-submucosa coated | 26.3 ± 4.06 |

**Table 4.**

| Effect of submucosal tissue co-culture on the proliferation of 3 human carcinoma cell lines | | |
|---|---|---|
| When cells reach approximately 50-60% confluence, submucosal tissue of about 1x1 cm (approximately 0.5g) was placed in direct contact with the cultures. Cell count was performed 3 days following co-culture. | | |

| | Control (x10⁴) at the day 4 | Co-culture |
|---|---|---|
| A549 Lung Ca | 45.0 ± 6.14 | all dead |
| MCF-7 breast Ca | 30.1 ± 5.20 | all dead |
| colon Ca | 54.2 ± 6.20 | all dead |

### Example 2

### The Effect of Submucosal Tissue on the Preimplantation Development of Mouse Embryos.

To determine the effect of submucosal tissue on the growth of undifferentiated cells, one-cell fertilized mouse oocytes were cultured in the presence of submucosal tissue using standard eukaryotic cell culture techniques known to those skilled in the art. The single cell fertilized oocytes were cultured with submucosal tissue using three different test procedures: 1) contact culture: fertilized oocytes were placed directly on the submucosal tissue membrane and therefore physically associated with the membrane; 2) indirect culture: fertilized oocytes and co-cultured submucosal tissue membrane were physically separated by a stainless steel mesh which allowed only nutrients to freely diffuse between the two compartments; 3) fertilized oocytes were co-cultured with different doses of solubilized submucosal tissue. AA-submucosa represents solubilized submucosal tissue that was dialyzed against a 0.5% chloroform in 0.1% acetic acid solution. PBS-submucosa represents solubilized submucosal tissue that was dialyzed against 0.5% chloroform in a phosphate buffered solution (PBS); 4) fertilized oocytes were cultured on culture dishes previously coated with either AA-submucosa or PBS-submucosa. Early embryonic development was examined every day for a total of 5 days and the percentage of blastocyst-formation was determined.

Results indicated that in comparison with the control group where 60% of fertilized oocytes developed into the blastocyst state, co-culturing oocytes with submucosal tissue arrested embryonic development at the 2-cell stage, irrespective of whether the fertilized oocytes are physically associated with the membrane (see Table 5). These 2-cell embryos were stained trypan blue positive, indicating the death of these cells. Thus these results demonstrate the toxic effect of the submucosal tissue on undifferentiated cells.

The toxic effect of submucosal tissue to the embryo cells cannot be attributed to the presence of peracetic acid in the cell cultures (peracetic acid is used to sterilize submucosal tissue) for two reasons: 1) before the co-culture, AA-submucosa membrane was soaked in a large quantity of culture medium for 24 hours which was followed by extensive washes; 2) co-culture of submucosal tissue with native submucosal tissue membrane also resulted in similar toxicity to the embryonic development (see Table 6).

When oocytes were co-culture in the presence of solubilized submucosal tissue (either by directly adding solubilized submucosal tissue to the culture medium or coating the culture dishes with submucosal tissue), there was only a slightly reduction in the blastocyst-forming rate (Table 7 and 8). This may not truly reflect the toxic effect of the submucosal tissue on undifferentiated cells since the solubilized submucosal tissue had been previously dialyzed in a dialysis-tubing with molecular weight cut-off (MWCO) of 10,000 Dalton and thus proteins smaller than 10,000 Dalton had been removed from the native tissue. It is possible that by using dialysis tubing of smaller MWCO, such as 1000 MWCO, one would be able to demonstrate a more obvious embryo-toxic effect of the solubilized submucosal tissue.

**Table 5.**

| The Effect of Submucosal Tissue Membrane on the Preimplantation Development of Mouse Embryos. | | | | | |
|---|---|---|---|---|---|
| | 1-cell | 2-cell | 8-cell | Blast. | Trypan Blue |
| Control | 15 | 100% | 93.3% | 60% | - |
| PA-Submucosa Indirect culture | 10 | 70% | 0 | 0 | + |
| PA-Submucosa Contact culture | 15 | 13% | 0 | 0 | + |

**Table 6.**

| The Effect of Submucosal Tissue Membrane on the Preimplantation Development of Mouse Embryos | | | |
|---|---|---|---|
| | 1-cell | Blast. | Trypan Blue |
| Control | 40 | 58% | - |
| PA-Submucosa (contact) | 40 | 0^{a} | + |
| PA-native (contact) | 20 | 0^{a} | + |

| | | | |
|---|---|---|---|
| ^{a}: Less than 20% developed to 2-cell stage | | | |

**Table 7.**

| The Effect of Fluidized Submucosal Tissue on the Preimplantation Development of Mouse Embryos. | | | | |
|---|---|---|---|---|
| | 1-cell | 2-cell | M | blast |
| Control | 15 | 93% | 93% | 87% |
| AA 200ul | 15 | 87% | 87% | 73% |
| AA-submucosa | 15 | 87% | 87% | 67% |
| AA 400ul | 15 | 93% | 93% | 46% |
| AA-submucosa | 15 | 93% | 87 % | 33% |
| AA800ul | 14 | 100% | 93% | 7% |
| AA-submucosa | 15 | 93% | 22% | 0 |

**Table 8.**

| The Effect of Fluidized Submucosal Tissue Coating on the Preimplantation Development of Mouse Embryos. | | | | |
|---|---|---|---|---|
| | 1-cell | 2-cell | M | Blast. |
| Control | 15 | 93% | 93% | 87% |
| AA coated | 9 | 100% | 89% | 89% |
| AA-submucosa | 10 | 90% | 70% | 70% |
| PBS coated | 7 | 100% | 86% | 71% |
| PBS-submucosa | 9 | 100% | 100% | 67% |

### Example 3

### Effect of Submucosal Tissue on the In vivo Growth of Tumor Cells

The effect of intestinal submucosal tissue on the growth of B16-F0 murine melanoma cells will be investigated in two different strains of mice, the BALB/c mouse and the athymic nude mouse. A total of seven groups (5 mice each) will be utilized in this investigation. 3x10⁶ cells will be injected subcutaneously into the backs of six groups of test mice. The control group of mice will be injected with sterile saline, the vehicle used to inject the tumor cells in the test mice. Two groups of the test animals will receive an additional injection of a suspension of submucosal tissue at the time the mice are injected with the tumor cells while the other groups will receive an additional injection of the submucosal tissue vehicle only. The submucosal tissue vehicle will be glycerol. When the first tumor mass is palpated at the injection site two of the remaining non-submucosal tissue treated test groups will be injected with a submucosal tissue suspension, while the other groups will be treated with only the submucosal tissue vehicle. One week later, a fifth group of mice (non-submucosal tissue treated) will be injected with the fluidized submucosal tissue composition, and one of the test groups that was administered fluidized intestinal submucosal tissue at the time of initial mass palpation will receive an additional treatment of submucosal tissue. One test group will be injected only with the submucosal tissue vehicle. Each of the groups will be compared for tumor size and degree of spread of the tumor cells (metastasis) for 28 days following the formation of a palpable tumor or until the cancer becomes terminal for the animal. Following death, the animals will be evaluated for tumor size, degree of local spread, and metastasis to the lung, liver, and kidneys. Length of survival time after administration of the tumor cells will also be evaluated.

## Claims

1. The use of submucosal tissue of a warm blooded vertebrate for the preparation of a pharmaceutical composition for inhibiting the growth of undifferentiated cells.

2. The use of submucosal tissue in accordance with claim 1 wherein the submucosal tissue comprises intestinal submucosa delaminated from both the tunica muscularis and at least the luminal portion of the tunica mucosa.

3. The use of submucosal tissue in accordance with claim 1 wherein the submucosal tissue is fluidized.

4. The use of submucosal tissue in accordance with claim 1 wherein the submucosal tissue is combined with a pharmaceutically acceptable carrier.

5. The use of submucosal tissue in accordance with claim 1 wherein the submucosal tissue is solubilized by digestion with an enzyme for a time sufficient to form a substantially homogeneous solution.

6. The use of submucosal tissue according to claim 1, in which the submucosal tissue is comminuted to form a suspension of submucosal tissue comprising tunica submucosa in an aqueous medium.

7. The use according to claim 1 in which the undifferentiated cells are neoplastic cells.

8. The use according to claim 1 in which the undifferentiated cells are tumor cells.

## Patentansprüche

1. Verwendung eines submukösen Gewebes eines warmblütigen Wirbeltieres zur Herstellung einer pharmazeutischen Zusammensetzung zur Inhibierung des Wachstums von undifferenzierten Zellen.

2. Verwendung eines submukösen Gewebes nach Anspruch 1, **dadurch gekennzeichnet, dass** das submuköse Gewebe intestinale Submukosa aufweist, die von sowohl der Tunica muscularis und wenigstens dem luminalen Bereich der Tunica mucosa abgelöst ist.

3. Verwendung von submukösem Gewebe nach Anspruch 1, **dadurch gekennzeichnet, dass** das submuköse Gewebe verflüssigt ist.

4. Verwendung von submukösem Gewebe nach Anspruch 1, **dadurch gekennzeichnet, dass** das submuköse Gewebe mit einem pharmazeutisch verträglichen Träger kombiniert ist.

5. Verwendung von submukösem Gewebe nach Anspruch 1, **dadurch gekennzeichnet, dass** das submuköse Gewebe durch Aufschließen mit einem Enzym für einen ausreichend langen Zeitraum zur Bildung einer im wesentlichen homogenen Lösung löslich gemacht ist.

6. Verwendung von submukösem Gewebe nach Anspruch 1, **dadurch gekennzeichnet, dass** das submuköse Gewebe zerkleinert ist, um eine Suspension von submukösem Gewebe enthaltend Tunika submucosa in einem wässrigen Medium zu bilden.

7. Verwendung nach Anspruch 1, **dadurch gekennzeichnet, dass** die undifferenzierten Zellen neoplastische Zellen sind.

8. Verwendung nach Anspruch 1, **dadurch gekennzeichnet, dass** die undifferenzierten Zellen Tumorzellen sind.

## Revendications

1. Utilisation de tissus sous-muqueux d'un vertébré à sang chaud pour la préparation d'une composition pharmaceutique destinée à inhiber la croissance de cellules indifférenciées.

2. Utilisation de tissus sous-muqueux selon la revendication 1, dans laquelle les tissus sous-muqueux comprennent des sous-muqueuses intestinales délaminées ou exfoliées à la fois de la tunique musculaire et au moins de la portion se rapportant à la lumière ou portion luminale de la tunique muqueuse.

3. Utilisation de tissus sous-muqueux selon la revendication 1, dans laquelle les tissus sous-muqueux sont fluidisés.

4. Utilisation de tissus sous-muqueux selon la revendication 1, dans laquelle les tissus sous-muqueux sont combinés avec un excipient pharmaceutiquement acceptable.

5. Utilisation de tissus sous-muqueux selon la revendication 1, dans laquelle les tissus sous-muqueux sont solubilisés par digestion avec une enzyme pendant une durée suffisante pour former une solution sensiblement homogène.

6. Utilisation de tissus sous-muqueux selon la revendication 1, dans laquelle les tissus sous-muqueux sont réduits en poudre pour former une suspension de tissus sous-muqueux comprenant une tunique sous-muqueuse dans un milieu aqueux.

7. Utilisation selon la revendication 1, dans laquelle les cellules indifférenciées sont des cellules neoplasiques.

8. Utilisation selon la revendication 1, dans laquelle les cellules indifférenciées sont des cellules tumorales.
